# EUROPÄISCHE PATENTSCHRIFT

(11) **EP 3 768 169 B1**
(45) Veröffentlichungstag und Bekanntmachung des Hinweises auf die Patenterteilung: **05.07.2023**
(21) Anmeldenummer: 19701848.4
(22) Anmeldetag: 25.01.2019
(51) Int. Cl.: A61B 8/00, G01S 7/52, G10K 11/34, A61B 8/08, G01S 15/89

(54) **ULTRASCHALLVORRICHTUNG ZUR MEDIZINISCHEN UNTERSUCHUNG MITTELS ULTRASCHALLWELLEN**
ULTRASONIC DEVICE FOR MEDICAL EXAMINATION BY MEANS OF ULTRASONIC WAVES
DISPOSITIF À ULTRASONS POUR L'ANALYSE MÉDICALE AU MOYEN D'ONDES ULTRASONORES

(30) Priorität: 21.03.2018 DE 102018204357
(43) Veröffentlichungstag der Anmeldung: 27.01.2021
(73) Patentinhaber: H-NEXT GMBH, 91052 Erlangen (DE)
(72) Erfinder: HEID, Oliver, 91052 Erlangen (DE)
(74) Vertreter: Fink Numrich Patentanwälte PartmbB
(86) Internationale Anmeldenummer: PCT/EP2019/051848
(87) Internationale Veröffentlichungsnummer: WO 2019/179675

(56) Entgegenhaltungen:
- US-A1- 2008 110 266
- US-A1- 2014 058 266

## Beschreibung

Die Erfindung betrifft eine Ultraschallvorrichtung zur medizinischen Untersuchung mittels Ultraschallwellen.

In der medizinischen Diagnostik werden häufig Ultraschallvorrichtungen verwendet, um menschliches bzw. tierisches Gewebe durch die Erfassung von Ultraschallechos zu untersuchen. Hierzu wird ein Schallkopf oberhalb des zu untersuchenden Gewebesektors angedrückt und über ein Array von elektroakustischen Wandlern Ultraschallwellen in den Gewebesektor gesendet. Mittels der Wandler werden die daraus resultierenden Ultraschallechos erfasst, wobei aus diesen Ultraschallechos ein Bild des Gewebesektors rekonstruiert wird, das je nach Anzahl und Anordnung der elektroakustischen Wandler zweidimensional oder dreidimensional sein kann.

Im Rahmen der Verarbeitung der erfassten Ultraschallechos werden die gewonnenen analogen Abtastwerte digitalisiert und einer sog. Strahlformung unterzogen, bei der die Abtastwerte der verschiedenen elektroakustischen Wandler geeignet zeitverschoben und addiert werden, um hierdurch die Position von Reflektoren in Richtung der Gewebetiefe sowie in Winkelrichtung relativ zu dem Array aus elektroakustischen Wandlern zu rekonstruieren. Üblicherweise erfolgt die Signalverarbeitung der über den Schallkopf erfassten Ultraschallechos in einer sperrigen Gerätebox, an der die analogen elektrischen Empfangssignale des Schallwandlers über ein Koaxialkabelbündel übertragen werden. Die Gerätebox kann z.B. kombiniert mit einer Tastatur und einer Anzeigeeinheit in einem Rollwagen eingebaut sein.

Die oben beschriebene Strahlformung wird derzeit synchron mit der Taktung des Analog-Digital-Konverters durchgeführt, mit dem die analogen Abtastwerte digitalisiert werden. Dabei besteht das Problem, dass im Rahmen eines Messvorgangs, der das Aussenden zumindest eines Ultraschallpulses sowie den Empfang seiner Echos über einen vorbestimmten Zeitraum umfasst, mittels der Strahlformung nur Gewebepositionen entlang eines Strahls in einer Winkelrichtung rekonstruiert werden können. Um somit ein Bild des gesamten Gewebesektors zu erhalten, müssen die gleichen Messvorgänge mehrmals wiederholt werden, wobei für jeden Messvorgang die Strahlformung entsprechend angepasst wird, um die Gewebepositionen in einer anderen Winkelrichtung zu rekonstruieren. Auf diese Weise erhält man einen Fächer von unterschiedlichen Winkelrichtungen, der das Bild ergibt. Zwar gibt es auch Ultraschallvorrichtungen, die mehrere Strahlformungs-Recheneinheiten parallel für unterschiedliche Winkelrichtungen verwenden. Dies erhöht jedoch die Komplexität der Ultraschallvorrichtung.

Zur Verkleinerung von Ultraschallvorrichtungen ist es aus dem Stand der Technik bekannt, in dem Schallkopf auch einen Teil der Signalverarbeitung zu integrieren, der üblicherweise in einer Gerätebox durchgeführt wird. Insbesondere ist es bekannt, einen Analog-Digital-Konverter in Kombination mit einer Strahlformungs-Recheneinheit in dem Schallkopf zu verbauen und anschließend die aus der Strahlformung gewonnenen Bilddaten über eine Datenschnittstelle an eine Bildformungs- und Anzeigeeinheit zu übergeben. Dabei besteht jedoch das Problem, dass die Strahlformungs-Recheneinheit eine hohe Rechenleistung benötigt und somit zu einer starken Erwärmung führt, die in Schallköpfen, welche in der Regel von Hand durch einen Benutzer geführt werden, nicht erwünscht ist. Als Konsequenz wird nur eine geringe Anzahl von elektroakustischen Wandlern in dem Schallkopf verbaut, um hierdurch die benötigte Rechenleistung für die Strahlformung zu begrenzen. Dies führt jedoch zu einer geringen Bildauflösung und geringen Bildwiederholraten. Darüber hinaus bleibt das Problem bestehen, dass ein Strahlformungsvorgang immer nur für eine Winkelrichtung des entsprechenden Gewebesektors durchgeführt werden kann.

Die Druckschrift US 2008/110266 A1 offenbart eine Ultraschallvorrichtung, bei der die mit einem Schallkopf erfassten Ultraschallechos digitalisiert werden und einer Vorverarbeitung unterzogen werden. Die vorverarbeiteten digitalisierten Daten werden über eine drahtlose Schnittstelle an eine Haupteinheit übermittelt, die unter anderem einen Puffer und einen Pixelformer umfasst. Der Pixelformer kann dabei digitale Strahlformung durchführen.

Das Dokument US 2014/058266 A1 beschreibt eine Ultraschallvorrichtung, bei der von einem Schallkopf stammende digitalisierte Rohdaten über ein Datennetz übertragen werden und anschließend einer digitalen Strahlformung unterzogen werden.

Aufgabe der Erfindung ist es, eine Ultraschallvorrichtung zur medizinischen Untersuchung mittels Ultraschallwellen zu schaffen, welche einfach aufgebaut und kostengünstig herzustellen ist und dabei Ultraschallbilder mit hoher Qualität liefert.

Diese Aufgabe wird durch die Ultraschallvorrichtung gemäß Patentanspruch 1 gelöst. Weiterbildung der Erfindung sind in den abhängigen Ansprüchen definiert.

Die erfindungsgemäße Ultraschallvorrichtung dient zur medizinischen Untersuchung des menschlichen oder tierischen Körpers. Hierzu umfasst die Ultraschallvorrichtung einen beweglichen Schallkopf, der an einem Körper eines Patienten positionierbar ist.

Der Patient kann dabei ein Mensch oder ein Tier sein. Vorzugsweise ist der bewegliche Schallkopf derart ausgestaltet, dass er durch die Hand eines Benutzers (insbesondere eines Arztes) gehalten und geführt werden kann. Hierdurch kann der Benutzer den Schallkopf an beliebige Positionen am Patientenkörper anordnen, um das darunter liegende Gewebe mittels Ultraschallwellen zu untersuchen.

Der bewegliche Schallkopf umfasst einen Wandler-Array aus elektroakustischen Wandlern, um Ultraschallsignale in den Körper zu senden und als analoge Rohdaten Ultraschallechos der gesendeten Ultraschallsignale zu empfangen. Vorzugsweise handelt es sich bei den elektroakustischen Wandlern um Piezoelemente. Je nach Ausgestaltung der Ultraschallvorrichtung kann die Anzahl der elektroakustischen Wandler variieren. Häufig werden Wandler-Arrays aus 128 elektroakustischen Wandlern verwendet. Ein jeweiliger Wandler kann dabei Ultraschallwellen durch Zuführung elektrischer Spannung in einem Sendebetrieb erzeugen. Ferner kann jeder Wandler auch Ultraschallwellen in einem Empfangsbetrieb erfassen, in dem keine elektrische Spannung an die jeweiligen Wandler angelegt wird.

Der bewegliche Schallkopf der erfindungsgemäßen Ultraschallvorrichtung umfasst einen Analog-Digital-Konverter, der dazu konfiguriert ist, in Abhängigkeit von den empfangenen analogen Rohdaten digitale Rohdaten zu generieren, wobei die digitalen Rohdaten Messdatensätze für zeitlich aufeinander folgende Messzeitintervalle umfassen. Ein jeweiliger Messdatensatz umfasst Ultraschallechos aus einem (zweidimensionalen und ggf. auch dreidimensionalen) Gewebesektor des Körpers, wobei die Ultraschallechos aus einem Sendevorgang von einem oder mehreren Ultraschallsignalen durch zumindest einen Wandler im Sendebetrieb resultieren. Die Ultraschallechos werden durch Abtastwerte für Abtastzeitpunkte des jeweiligen Messzeitintervalls für eine Mehrzahl von Empfangskanälen aus jeweils zumindest einem Wandler im Empfangsbetrieb repräsentiert. In einer bevorzugten Variante der Erfindung entspricht die Anzahl der Empfangskanäle der Anzahl an Wandlern, d.h. jeder Wandler stellt einen Empfangskanal dar, über den im Empfangsbetrieb ein entsprechender Abtastwert aufgrund von reflektierten Ultraschallechos erfasst wird. In einer Variante der Erfindung wird im Rahmen des Sendevorgangs nur ein einzelner Wandler zum Aussenden von Ultraschallechos genutzt. In der Regel werden über die aufeinander folgenden Messzeitintervalle unterschiedliche Wandler zum Senden genutzt. Üblicherweise werden nach dem Sendevorgang alle Wandler des Arrays in den Empfangsbetrieb geschaltet.

Die erfindungsgemäße Ultraschallvorrichtung umfasst ferner eine digitale Datenschnittstelle, über die der Schallkopf mit einer separaten, nicht zum Schallkopf gehörigen Rechnereinrichtung gekoppelt ist. Mit anderen Worten ist die Rechnereinrichtung ein von dem beweglichen Schallkopf separiertes Bauteil. Der Schallkopf ist ferner derart konfiguriert, dass er die digitalen Rohdaten, vorzugsweise direkt ohne Pufferung, über die digitale Datenschnittstelle sendet. Mit anderen Worten werden die digitalen Rohdaten mittels des Schallkopfs über die digitale Datenschnittstelle gestreamt. Vorzugsweise liegt die Datenrate zum Senden der Rohdaten über diese Datenschnittstelle bei mindestens 1 GB/s.

Die separate Rechnereinrichtung, welche Bestandteil der erfindungsgemäßen Ultraschallvorrichtung ist, umfasst einen Rohdaten-Pufferspeicher, in dem die jeweiligen Messdatensätze der digitalen Rohdaten gepuffert werden. Mit anderen Worten ist jeder über die digitale Datenschnittstelle übertragene Messdatensatz für eine bestimmte Zeitspanne in dem Rohdaten-Pufferspeicher zwischengespeichert, wobei die Zeitspanne von der Größe des Pufferspeichers abhängt. In einer bevorzugten Variante wird als Pufferspeicher ein sog. Ringpuffer genutzt.

Die separate Rechnereinrichtung ist ferner derart ausgestaltet, dass sie für die gepufferten Messdatensätze jeweils eine digitale Strahlformung durch zeitverzögerte Addition von Abtastwerten durchführt, um Bildwerte von einer Vielzahl von Gewebepositionen in verschiedenen Gewebetiefen und mit mehreren Gewebepositionen für jede Gewebetiefe, d.h. für unterschiedliche Winkelrichtungen und unterschiedliche Gewebetiefen, zu ermitteln. Vorzugsweise decken die Gewebepositionen den gesamten Gewebesektor ab. Auf diese Weise wird ein rekonstruiertes Bild des Gewebesektors erhalten. Je nach Ausgestaltung des Schallkopfs kann dabei z.B. ein zweidimensionales oder dreidimensionales Bild rekonstruiert werden.

Mit anderen Worten läuft die digitale Strahlformung aufgrund der Verwendung des Rohdaten-Pufferspeichers entkoppelt von der Taktung des Analog-Digital-Konverters und somit asynchron zu dieser Taktung ab. Dabei kann die digitale Strahlformung aufgrund der Pufferung der jeweiligen Messdatensätze mehrmals für den entsprechenden Messdatensatz für unterschiedliche Winkelrichtungen durchgeführt werden, so dass mit einem Messdatensatz ein rekonstruiertes Bild des gesamten Gewebesektors erzeugt werden kann.

Die in der erfindungsgemäßen Ultraschallvorrichtung verwendete Rechnereinrichtung ist ferner derart ausgestaltet, dass sie basierend auf den rekonstruierten Bildern einen Bildstrom aus zeitlich aufeinander folgenden rekonstruierten Bildern oder aus daraus berechneten Bildern mit einer vorgegebenen Bildwiederholrate generiert und einem Anzeigemittel (insbesondere einem Display) zuführt, das den Bildstrom wiedergibt. Mit anderen Worten kann die Wiedergabe der Bilder geeignet auf die Dauer der Strahlformung abgestimmt werden, so dass ein Bildstrom mit einer gewünschten Bildwiederholrate erzeugt und angezeigt werden kann. Das Anzeigemittel ist dabei Bestandteil der erfindungsgemäßen Ultraschallvorrichtung.

Die erfindungsgemäße Ultraschallvorrichtung weist den Vorteil auf, dass durch die Entkopplung der Strahlformung von der Digitalisierung der Rohdaten keine besonderen Echtzeit-Anforderungen an die Rechnereinrichtung gestellt werden müssen. Es können somit handelsübliche PC-Komponenten für die Rechnereinrichtung genutzt werden, wobei der Vorgang der Strahlformung und der Erzeugung des Bildstroms durch Software realisiert werden kann, die auf einem Prozessor eines handelsüblichen PC-Motherboards läuft. Darüber hinaus wird durch die erfindungsgemäße Ultraschallvorrichtung sichergestellt, dass der Vorgang der Strahlformung nicht im Schallkopf durchgeführt wird, wodurch Anforderungen an eine möglichst geringe Wärmeentwicklung im Schallkopf erfüllt werden können.

In einer bevorzugten Variante der erfindungsgemäßen Ultraschallvorrichtung wird als Anzeigemittel ein synchronisierbares Display verwendet, dessen Bildwiederholrate variiert werden kann. Auf diese Weise können etwaige Schwankungen in der Bildwiederholrate des generierten Bildstroms ausgeglichen werden und hierdurch Bildartefakte vermieden werden, die bei nicht-synchronisierbaren Displays auftreten können. Beispiele von synchronisierbaren Displays sind unter dem Namen AMD Freesync oder Nvidia G-Sync bekannt.

In einer besonders bevorzugten Ausgestaltung der erfindungsgemäßen Ultraschallvorrichtung ist der Rohdaten-Pufferspeicher zur gleichzeitigen Pufferung einer Vielzahl von aufeinander folgenden Messdatensätzen eingerichtet, d.h. es werden gleichzeitig mehrere Messdatensätze in dem Rohdaten-Pufferspeicher vorgehalten. Vorzugsweise wird diese Variante mit einer Rechnereinrichtung kombiniert, welche derart ausgestaltet ist, dass sie für mehrere der Vielzahl von gepufferten Messdatensätzen zeitlich parallel jeweilige rekonstruierte Bilder ermittelt. Auf diese Weise kann die Ermittlung der rekonstruierten Bilder beschleunigt werden und somit eine höhere Bildwiederholrate erreicht werden.

In einer weiteren bevorzugten Variante der erfindungsgemäßen Ultraschallvorrichtung ist die Rechnereinrichtung derart ausgestaltet, dass die Bilder des Bildstroms jeweils als eine Mittelung aus mehreren zeitlich aufeinander folgenden rekonstruierten Bildern berechnet werden. Hierdurch kann die Bildqualität verbessert werden.

In einer weiteren bevorzugten Variante liegt die vorgegebene Bildwiederholrate des Bildstroms bei 50 Hz oder mehr, z.B. bei 60 Hz oder 75 Hz. Bei diesen Frequenzen ist sichergestellt, dass die Bilder des Bildstroms für das menschliche Auge ineinander verschmelzen, so dass kein Flimmern bzw. Flackern wahrgenommen wird.

In einer weiteren bevorzugten Variante der erfindungsgemäßen Ultraschallvorrichtung ist die Rechnereinrichtung ferner derart ausgestaltet, dass sie digitale Steuerdaten ermittelt, basierend auf denen der Betrieb der Wandler des Wandler-Arrays zum Senden von Ultraschallsignalen und zum Empfangen von Ultraschallechos für die aufeinander folgenden Messzeitintervalle festgelegt ist. Die Rechnereinrichtung umfasst dabei einen Steuerdaten-Pufferspeicher (vorzugsweise einen Ringpuffer), in dem die digitalen Steuerdaten gepuffert werden. Ferner ist der Schallkopf derart ausgestaltet, dass er über die digitale Datenschnittstelle oder eine weitere digitale Datenschnittstelle zwischen Schallkopf und Rechnereinrichtung die digitalen Steuerdaten, vorzugsweise direkt ohne Pufferung, ausliest und basierend auf diesen ausgelesenen Steuerdaten die Wandler des Wandler-Arrays ansteuert.

Die Steuerdaten werden somit vorab mit der Rechnereinrichtung ermittelt und dann im Rahmen der Ansteuerung der Wandler genutzt. Durch die Verwendung des Steuerdaten-Pufferspeichers ist dabei die Generierung der Steuerdaten entkoppelt von deren Verwendung im Schallkopf. Auf diese Weise wird es wiederum möglich, die Steuerdaten durch eine Rechnereinrichtung ohne besondere Echtzeit-Anforderungen zu generieren. Insbesondere kann zur Erzeugung der Steuerdaten ein herkömmlicher PC verwendet werden, auf dessen Prozessor eine Software läuft, welche die Steuerdaten erzeugt. Vorzugsweise liegt die Datenrate zum Auslesen der Steuerdaten über die entsprechende digitale Datenschnittstelle bei mindestens 1 GB/s.

Je nach Ausgestaltung kann die digitale Datenschnittstelle eine drahtgebundene Datenschnittstelle und/oder eine drahtlose Datenschnittstelle umfassen bzw. darstellen. In gleicher Weise kann auch die obige weitere digitale Datenschnittstelle ausgestaltet sein. In einer bevorzugten Ausführungsform umfasst bzw. ist die digitale Datenschnittstelle und/oder die weitere digitale Datenschnittstelle eine an sich bekannte PCI-Express-Schnittstelle. Über eine solche Schnittstelle können hohe Datenraten erreicht werden. Der PCI Express Standard ist hinlänglich aus dem Stand der Technik bekannt und die PCI-Express-Schnittstelle ist dabei nicht auf eine bestimmte Version des Standards bzw. der dabei verwendeten Datenleitungen beschränkt.

In einer weiteren bevorzugten Ausgestaltung umfasst der Schallkopf der erfindungsgemäßen Ultraschallvorrichtung einen Hochspannungssender, um Hochspannungssignale zu erzeugen, die dem Wandler-Array zur Erzeugung von Ultraschallwellen durch einen oder mehrere Wandler im Sendebetrieb über ein Weichenarray aus Sende-Empfangs-Weichen zugeführt werden, wobei die Wandler des Wandler-Arrays durch das Weichen-Array in den Sendebetrieb oder den Empfangsbetrieb geschaltet werden können. Wird in dieser Variante die oben beschriebene Pufferung der Steuerdaten verwendet, so werden die Steuerdaten durch den Hochspannungssender verarbeitet, der basierend auf diesen Steuerdaten entsprechende Hochspannungssignale generiert. Ferner bewirken die Steuerdaten, dass das Weichen-Array derart geschaltet wird, dass alle Wandler im Empfangsbetrieb von dem Hochspannungssender entkoppelt sind.

In einer weiteren Ausgestaltung der erfindungsgemäßen Ultraschallvorrichtung umfasst der Schallkopf einen Vorverstärker, der dazu eingerichtet ist, die analogen Rohdaten vor der Zuführung zum Analog-Digital-Konverter zu verstärken. Der Analog-Digital-Konverter digitalisiert in diesem Fall die verstärkten Rohdaten.

Ein Ausführungsbeispiel der Erfindung wird nachfolgend anhand der beigefügten Figuren detailliert beschrieben.

Es zeigen:
- Fig. 1: eine schematische Darstellung einer medizinischen Ultraschallvorrichtung gemäß dem Stand der Technik; und
- Fig. 2: eine schematische Darstellung einer Ausführungsform einer erfindungsgemäßen Ultraschallvorrichtung.

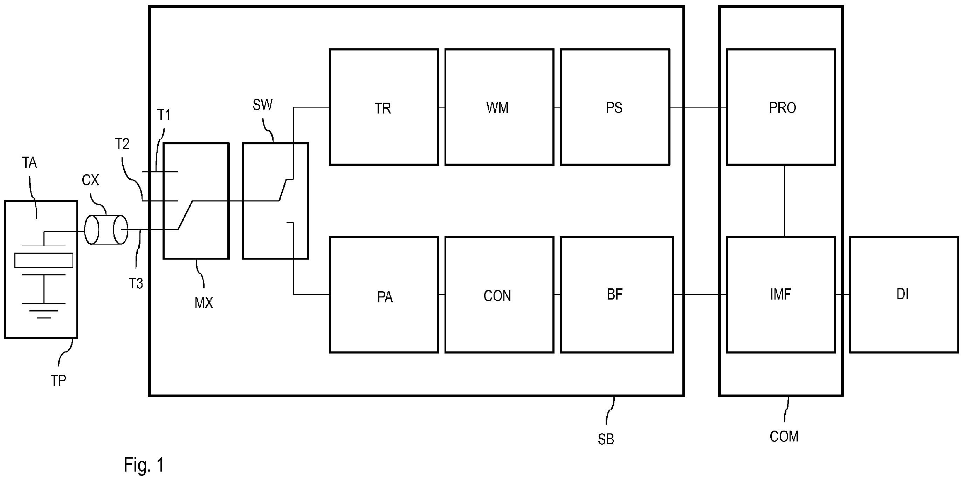

Bevor eine Ausführungsform einer erfindungsgemäßen Ultraschallvorrichtung beschrieben wird, erfolgt zunächst die Beschreibung des Aufbaus einer an sich bekannten Ultraschallvorrichtung, die in Fig. 1 gezeigt ist.

Die Ultraschallvorrichtung gemäß Fig. 1 umfasst einen bewegbarer Schallkopf TP (TP = Transducer Probe), der durch die Hand eines Benutzers geführt und auf die Haut eines Patienten gesetzt werden kann, um mittels Ultraschall die unter der Haut liegenden Körperteile zu untersuchen. Hierzu enthält der Schallkopf einen Wandler-Array TA aus einer Vielzahl von elektroakustischen Wandlern (z.B. 128 Wandler), wobei dieses Array lediglich schematisch durch die Darstellung eines einzelnen Wandlers angedeutet ist. In der Regel sind die elektroakustischen Wandler als Piezoelemente ausgestaltet, über welche durch Anlegen einer Hochspannung Ultraschallsignale ausgesendet werden können. Ebenso können über die Piezoelemente entsprechende Ultraschallechos empfangen werden. Die Ultraschallsignale werden dabei als Schallpulse mit einer Wiederholrate von mehreren kHz emittiert.

Das Schalten zwischen einem Sendebetrieb und einem Empfangsbetrieb eines entsprechenden Piezoelements bzw. Wandlers wird über ein Weichen-Array aus Sende-Empfangs-Weichen ermöglicht, welches in Fig. 1 mit Bezugszeichen SW bezeichnet ist und weiter unten näher erläutert wird. Aus Übersichtlichkeitsgründen ist das Weichen-Array lediglich durch eine Weiche angedeutet.

Der bewegliche Schallkopf TP ist über ein Koaxialkabelbündel CX an eine Gerätebox SB angeschlossen, welche im Folgenden auch als Scannerbox bezeichnet wird. Bei dieser Gerätebox handelt es sich um ein sperriges Bauteil, das die wesentlichen Komponenten zur Signalverarbeitung der über den Schallkopf TP empfangenen Ultraschallechos beinhaltet. An die Gerätebox ist ein Rechner bzw. Computer COM in der Form eines PCs angeschlossen, der wiederum mit einem Display DI verbunden ist. In der Regel bilden die Scannerbox, der Computer und das Display eine Einheit, die z.B. in einem Rollwagen integriert ist. Darüber hinaus kann der Computer auch Bestandteil der Gerätebox sein.

Die Scannerbox SB umfasst drei Anschlüsse T1, T2 und T3, die an einen Multiplexer MX gekoppelt sind, über den einer dieser Anschlüsse ausgewählt werden kann. An jeden Anschluss kann ein separater Schallkopf über ein entsprechendes Koaxialkabelbündel angeschlossen werden. Über den Multiplexer MX wird derjenige Schallkopf ausgewählt, der zur Erfassung der Ultraschallechos aktuell genutzt werden soll. In Fig. 1 ist lediglich ein einzelner Schallkopf TP gezeigt, der an dem Anschluss T3 angeschlossen ist und dort über den Multiplexer MX mit den weiteren Komponenten der Scannerbox SB verbunden ist.

An den Multiplexer MX schließt sich das bereits erwähnte Weichen-Array SW an, wobei dieses Array mehrere Sende-Empfangs-Weichen umfasst, deren Anzahl der Anzahl von Wandlern in den Wandler-Array TA entspricht. Mit anderen Worten ist für jeden Wandler eine Sende-Empfangs-Weiche vorgesehen. Durch Schalten einer entsprechenden Sende-Empfangs-Weiche in den Sendebetrieb wird der zugeordnete Wandler mit einem Hochspannungssender TR verbunden, über den durch Zuführung entsprechender Hochspannungssignale zum Wandler Ultraschallwellen erzeugt werden. Ist demgegenüber die entsprechende Weiche im Empfangsbetrieb geschaltet, werden vom Schallkopf TP erfasste Abtastwerte von Ultraschallechos auf einen Empfangspfad umfassend einen Vorverstärker PR, einen Analog-Digital-Konverter CON und einen Strahlformer BF gegeben.

Üblicherweise werden den Wandlern mittels des Hochspannungssenders TR Spannungen zwischen ±10 V bis ±100V zugeführt. Der Hochspannungssender ist dabei mit einem Wellenformspeicher WM und einem sog. Puls-Sequencer PS verbunden. Der Puls-Sequencer stellt eine Ablaufsteuerung dar, mit der das Senden von Ultraschallpulsen und das Empfangen von Ultraschallechos in entsprechenden Messzeitintervallen festgelegt werden. Für auszusendende Ultraschallpulse liest der Puls-Sequencer die Wellenform des entsprechen Pulses aus dem Wellenformspeicher in der Form eines Spannungsmusters aus Hochspannungen aus. Dieses Spannungsmuster wird dem Hochspannungssender zur Generierung der entsprechenden Hochspannungen zugeführt. Darüber hinaus steuert der Puls-Sequencer die Weichen des Weichen-Arrays SW, um hierdurch die jeweiligen Weichen gemäß dem gewünschten Messablauf in den Sende- bzw. Empfangszustand zu schalten. Der Puls-Sequencer PS ist beispielsweise als FPGA (FPGA = Field Programmable Gate Array) ausgestaltet. Der Puls-Sequencer ist mit dem Prozessor PRO (d.h. der CPU) des Computers COM verbunden. Über den Prozessor werden Kommandos zur Durchführung von Ultraschallmessungen an den Puls-Sequencer gegeben.

Die durch den Schallkopf TP empfangenen Ultraschallechos werden über den Vorverstärker PA (PA = Pre-Amplifier) verstärkt. Der Verstärker enthält dabei für jeden einzelnen Wandler ein Vorverstärkerelement, das die Empfangssignale des entsprechenden Wandlers verstärkt. Die im Vorverstärker PA empfangenen Daten stellen analoge Rohdaten dar, welche aufeinander folgende Messdatensätze enthalten. Ein jeweiliger Messdatensatz bezieht sich dabei auf eine einzelne, mit dem Schallkopf TP durchgeführte Messung. Bei einer solchen einzelnen Messung werden gemäß einem vorbestimmten Schema ein oder mehrere Ultraschallsignale durch einen oder mehrere Wandler des Wandler-Arrays TA ausgesendet und anschließend werden innerhalb eines vorbestimmten Messzeitintervalls die Ultraschallechos erfasst, die durch Reflexionen in dem Gewebesektor hervorgerufen werden, in den die Ultraschallsignale durch den Schallkopf TP eingestrahlt wurden. Über die Länge des Messzeitintervalls kann dabei die Gewebetiefe festgelegt werden, bis zu der Ultraschallechos empfangen und weiterverarbeitet werden sollen.

Üblicherweise werden nach dem Aussenden von Ultraschallsignalen alle Wandler mittels des Weichen-Arrays SW in den Empfangsbetrieb geschaltet. Demzufolge werden für jeden Wandler Abtastwerte erhalten, die für eine Vielzahl von Abtastzeitpunkten entsprechend einer Abtastfrequenz innerhalb des Messzeitintervalls erhalten werden. Für ein jeweiliges Messzeitintervall umfassen die Rohdaten somit einen entsprechenden Messdatensatz, der für eine Vielzahl von Abtastzeitpunkten jeweils Abtastwerte für alle Wandler umfasst, wobei ein jeweiliger Abtastwert das empfangene Ultraschallecho zu einem Abtastzeitpunkt repräsentiert.

Die verstärkten analogen Rohdaten werden einem Analog-Digital-Konverter CON zugeführt, der für die Abtastwerte jedes elektroakustischen Wandlers ein A/D-Wandlerelement umfasst. In den einzelnen A/D-Wandlerelementen werden die verstärkten Abtastwerte digitalisiert. Der Analog-Digital-Konverter CON ist mit einer vorgegebenen Frequenz getaktet und erzeugt eine der Anzahl von elektroakustischen Wandlern entsprechende Anzahl von digitalen Datenströmen, die Ultraschallechos aus zunehmender Gewebetiefe für jeweilige Messzeitintervalle darstellen. Die digitalen Datenströme des Analog-Digital-Konverters werden anschließend einer Strahlformungs-Recheneinheit BF (BF = Beam Forming) zugeführt, die im Folgenden auch als Strahlformer bezeichnet wird und beispielsweise als FPGA ausgestaltet ist. In diesem Strahlformer werden in an sich bekannter Weise die Abtastwerte der unterschiedlichen elektroakustischen Wandler nach Art eines Phased-Arrays derart zeitverzögert, dass die zeitverzögerten Abtastwerte den Empfang eines Ultraschallechos ausgehend vom gleichen Reflexionspunkt im Gewebe darstellen. Die entsprechend zeitverzögerten Abtastwerte werden addiert und repräsentieren eine Position im untersuchten Gewebe in einer vorbestimmten Winkelrichtung und in einer vorbestimmten Gewebetiefe ausgehend von dem Wandler-Array. Die entsprechende Position stellt in dem später erzeugten Ultraschallbild einen Bildpunkt dar.

Als Ergebnis der im Strahlformer BF durchgeführten Strahlformung erhält man schließlich für jedes Messzeitintervall Bildpunktwerte (d.h. die Summe entsprechend zeitverzögerter Abtastwerte), und zwar für unterschiedliche Zeitpunkte und damit unterschiedliche Gewebetiefen, jedoch nur in einer einzelnen Winkelrichtung. Dies liegt daran, dass die Strahlformung des Strahlformers BF synchron mit der Erzeugung der digitalen Daten durch den Analog-Digital-Konverter CON durchgeführt wird, so dass der Strahlformer immer nur eine Zeitverzögerung für die jeweiligen Abtastwerte einstellen und somit nur Bildpunktwerte für eine Winkelrichtung ermitteln kann. Demzufolge ist es zur Rekonstruktion eines zweidimensionalen und ggf. auch dreidimensionalen Bilds des gesamten Gewebesektors erforderlich, dass mehrmalig hintereinander die gleiche Messung durch Aussenden entsprechender Ultraschallsignale durchgeführt wird, wobei für jede Messung die Strahlformung durch Anpassung der Zeitverzögerung für eine unterschiedliche Winkelrichtung durchgeführt wird. Optional besteht auch die Möglichkeit, mehrere Strahlformer BF parallel in der Gerätebox SB zu verbauen, was jedoch erheblichen Mehraufwand und erhebliche Mehrkosten verursacht.

Die durch den Strahlformer BF erhaltenen Bildpunktwerte werden schließlich zu dem Computer COM übertragen, der in an sich bekannter Weise mittels einer Bildformungs-Einheit IMF (IMF = Image Former) ein entsprechendes Bild auf dem Display DI generiert. Die Funktion der Bildformungs-Einheit wird in der Regel durch den Graphikchip des Computers COM realisiert, der ggf. auch Bestandteil des zentralen Prozessors PRO sein kann. Üblicherweise werden für die Darstellung auf dem Display DI Bilder verwendet, die eine Mittelung von mehreren aufeinander folgenden Bildern darstellen, die über die Strahlformung erhalten wurden. Die Bildwiederholrate des Displays DI ist dabei fest vorgegeben, so dass die dargestellte Bildfolge unter Bildartefakten, wie Jitter und Tearing (Umschaltung zwischen Bildern während des Bildaufbaus), leiden kann.

Die Schallgeschwindigkeit im biologischen Gewebe beträgt etwa 1450 m/s, so dass ein Ultraschallecho aus z.B. 15 cm Tiefe eine Laufzeit von ca. 200 µm besitzt. Somit können für diese Gewebetiefe pro Sekunde 5000 Messungen durchgeführt werden. Eine Datenerfassung für einen Gewebesektor mit z.B. 125 Winkelrichtungen dauert damit abhängig von der eingestellten maximalen Messtiefe, der Anzahl an Winkelrichtungen und der Anzahl an zur Mittelung herangezogenen Bildern zwischen z.B. 1 ms und mehr als 100 ms.

Wie sich aus der obigen Beschreibung ergibt, hat die herkömmliche Ultraschallvorrichtung gemäß Fig. 1 den Nachteil, dass mehrere Messungen benötigt werden, um ein Bild eines Gewebesektors mittels Strahlformung zu rekonstruieren. Dies liegt daran, dass die mit der Taktung des Analog-Digital-Konverters synchronisierte Strahlformung pro Messung immer nur Bildpunkte aus einer Winkelrichtung berechnen kann. Zwar können zur Umgehung dieses Problems mehrere Strahlformer eingesetzt werden, was jedoch sehr aufwändig und kostenintensiv ist. Darüber hinaus können bei der Anzeige des Bildstroms Bildartefakte auftreten. Ferner sind die Abmessungen der Ultraschallvorrichtung sehr groß, da zur Signalverarbeitung eine Gerätebox in Kombination mit einem Computer benötigt wird.

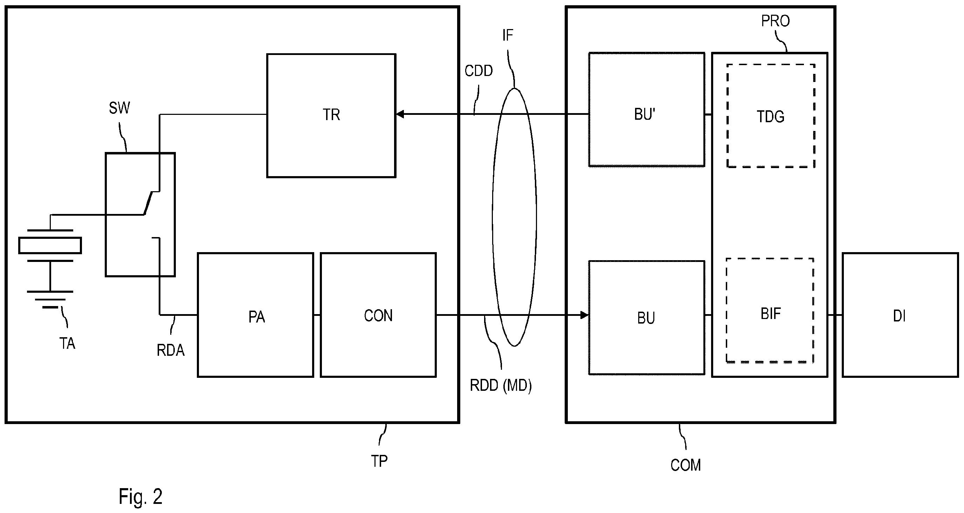

Die obigen Nachteile werden durch die in Fig. 2 gezeigte Ausführungsform einer erfindungsgemäßen Ultraschallvorrichtung behoben. Diese Ultraschallvorrichtung umfasst genauso wie die Vorrichtung der Fig. 1 einen beweglichen Schallkopf TP mit einem Wandler-Array TA aus elektroakustischen Wandlern. Analog zum Schallwandler der Fig. 1 wird auch der Schallwandler der Fig. 2 händisch an den zu untersuchenden Patientenkörper angedrückt, um mittels Ultraschallwellen das Gewebe unter der Andrückposition des Schallwandlers zu untersuchen.

Im Unterschied zum Schallkopf der Fig. 1 enthält der Schallkopf der Fig. 2 neben dem Wandler-Array TA weitere Komponenten, die in der Ausführungsform der Fig. 1 Teil der Scannerbox SB sind. Es handelt sich hierbei um das Weichen-Array SW, den Sender TR sowie den Vorverstärker PA und den Analog-Digital-Konverter CON. Diese Komponenten funktionieren analog wie in Fig. 1, so dass sie nicht nochmals im Detail beschrieben werden. Im Besonderen werden die über das Weichen-Array ausgegebenen analogen Rohdaten, die in Fig. 2 mit RDA bezeichnet sind, zunächst über den Vorverstärker PA verstärkt und dann in dem Analog-Digital-Konverter CON digitalisiert.

Erfindungswesentlich ist nunmehr, dass die durch den Analog-Digital-Konverter erzeugten digitalen Daten nicht unmittelbar einer Strahlformung unterzogen werden, sondern zunächst gepuffert werden, um die Strahlformung anschließend asynchron zur Taktung des Analog-Digital-Konverters auf den gepufferten Daten durchzuführen. Um dies zu ermöglichen, werden die von dem Analog-Digital-Konverter CON ausgegebenen digitalen Rohdaten direkt (d.h. ohne Pufferung) auf eine digitale Datenschnittstelle IF gegeben, welche die Daten in den Pufferspeicher BU schreibt, der vorzugsweise ein Ringpuffer ist. Die über die digitale Datenschnittstelle IF übertragenen Rohdaten sind in Fig. 2 mit dem Bezugszeichen RDD bezeichnet. Diese Rohdaten enthalten jeweilige Messdatensätze für entsprechende Messzeitintervalle, wobei die Struktur der Messdatensätze bereits oben erläutert wurde. Die Messdatensätze sind in Fig. 2 allgemein mit dem Bezugszeichen MD bezeichnet. Der Pufferspeicher BU ist Bestandteil einer separaten Rechnereinrichtung COM, die vorzugsweise ein herkömmlicher PC ist. Die Rechnereinrichtung ist somit eine separate Einheit, die nicht zu dem Schallkopf gehört.

Als digitale Datenschnittstelle IF wird in der hier beschriebenen Ausführungsform eine bidirektionale Schnittstelle basierend auf dem Standard PCI Express verwendet. Sofern die Datenrate der durch den Analog-Digital-Konverter ausgegebenen Rohdaten erreicht wird, kann jede Version dieses Standards eingesetzt werden, wobei derzeit die Versionen 1.0/1.1, 2.0/2.1, 3.0/3.1, 4.0 und 5.0 bekannt sind. Ebenso kann die Anzahl der zur Datenübertragung genutzten Leitungen verschieden gewählt werden, wobei derzeit die Verwendung von einer, zwei, vier, acht und sechszehn Leitungen bekannt ist. Beispielsweise kann als Datenschnittstelle PCIe3 x 16 (PCI Express Version 3.0/3.1 mit 16 Leitungen) bzw. PCIe2 x 8 (PCI Express Version 2.0/2.1 mit acht Leitungen) verwendet werden. In der hier beschriebenen Ausführungsform wird die PCI-Express-Schnittstelle auch zu der weiter unten beschriebenen Übertragung von digitalen Steuerdaten CDD von dem Computer COM hin zu dem Hochspannungssender TR genutzt. Der Datenübertragungsmechanismus über die Schnittstelle IF hat direkte Lese- und Schreibfähigkeit in den Pufferspeicher BU und auch in den weiter unten beschriebenen Pufferspeicher BU'. Auf diese Weise hängen die Datenübertragungsraten sowie die Übertragungslatenz nicht von Antwortzeiten des Betriebssystems der Rechnereinrichtung COM ab.

Der Pufferspeicher BU hat eine Größe, so dass eine Vielzahl von aufeinander folgenden Messdatensätzen MD gleichzeitig darin gespeichert ist. Die gespeicherten Messdatensätze werden dabei blockweise verarbeitet. Im Rahmen dieser Verarbeitung wird mit einer auf dem Prozessor PRO der Rechnereinrichtung COM laufenden Software BIF (BIF = Beam and Image Former) eine Strahlformung sowie die Erzeugung von rekonstruierten Bildern zur Anzeige auf dem Display DI durchgeführt.

Die Strahlformung erfolgt nach dem gleichen Prinzip wie in dem Strahlformer BF der Fig. 1, jedoch kann im Rahmen der Strahlformung mehrmals auf die Abtastwerte der jeweiligen gepufferten Messdatensätze zurückgegriffen werden, da diese für einen gewissen Zeitraum in dem Pufferspeicher BU gepuffert sind. Demzufolge wird im Rahmen der Strahlformung für einen jeweiligen Messdatensatz ein rekonstruiertes Bild des gesamten Gewebesektors mit einer Vielzahl von unterschiedlichen Winkelrichtungen und Gewebetiefen erhalten. Mit anderen Worten wird somit nur ein einziger Messdatensatz zur Rekonstruktion eines Bilds des gesamten Gewebesektors benötigt, wohingegen in der Ultraschallvorrichtung der Fig. 1 mehrere Messdatensätze erforderlich sind, um ein rekonstruiertes Bild des Gewebesektors zu erhalten.

Die mittels der Strahlformung rekonstruierten Bilder werden anschließend mit der Software BIF in einen Bildstrom gewandelt. Vorzugsweise werden dabei mehrere zeitlich aufeinander folgende rekonstruierte Bilder gemittelt und als ein einzelnes Bild auf dem Display DI wiedergegeben. Die auf dem Display DI wiedergegebene Bilderfolge hat vorzugsweise eine Bildwiederholfrequenz, die oberhalb der Flickerfrequenz des menschlichen Auges liegt, z.B. eine Bildwiederholfrequenz von 60 Hz oder 75 Hz. Als Display DI wird ein synchronisierbares Display verwendet, dessen Bildwiederholrate angepasst werden kann. Hierdurch können kurzfristige begrenzte Schwankungen der Bildrate des Bildstroms ausgeglichen werden. Solche synchronisierbaren Displays sind an sich bekannt. Beispielsweise handelt es sich um Displays vom Typ AMD Freesync oder Nvidia G-Sync.

Die Ultraschallvorrichtung der Fig. 2 unterscheidet sich von der Vorrichtung der Fig. 1 ferner dahingehend, dass die Steuerdaten CDD, über welche das Senden und Empfangen der elektroakustischen Wandler für die aufeinander folgenden Messungen eingestellt werden, auch in der Rechnereinrichtung COM generiert werden. Hierzu wird die Software TDG (TDG = Transmit Data Generator) genutzt, die auf dem Prozessor PRO der Rechnereinrichtung COM läuft. Diese Steuerdaten werden in einem Pufferspeicher BU' gepuffert, der wie der Pufferspeicher BU als Ringpuffer ausgestaltet sein kann. Die Steuerdaten CDD werden aus dem Puffer BU' durch den Hochspannungssender TR direkt ohne weitere Pufferung über die Schnittstelle IF ausgelesen. Gemäß den Steuerdaten erzeugt der Hochspannungssender dann die Hochspannungen, die zum Aussenden eines Ultrasignals durch die jeweiligen Wandler im Sendebetrieb benötigt werden. Die Steuerdaten spezifizieren auch, welche Wandler zu welchen Zeitpunkten im Sendebetrieb bzw. Empfangsbetrieb laufen sollen. Gemäß diesen Informationen werden dann die Weichen des Weichen-Arrays SW durch Signale des Hochfrequenzsenders TR geschaltet. Durch die Verwendung des Puffers BU' ist die Übertragung der Steuerdaten CDD nicht mehr abhängig von Antwortzeiten des Betriebssystems der Rechnereinrichtung COM.

Eine übliche Ausgestaltung einer Ultraschallvorrichtung umfasst 128 elektroakustische Wandler, wobei beispielsweise ein Abtastwert der Ultraschallechos von 2 Byte und eine Abtastrate von 15 MHz für ein Messzeitintervall von 200 µs verwendet werden. Dies führt zu einer Datenmenge von 750 kB und somit zu einer Datenrate von ca. 4 GB/s. Demzufolge sollte in einem solchen Fall die Datenrate der Datenschnittstelle IF in Richtung hin zum Puffer BU bei 4 GB/s oder mehr liegen. Bei der Speicherung von 125 Messdatensätzen muss der Pufferspeicher BU dabei mehrere 100 MB groß sein. Eine übliche Abtastrate zum Ansteuern der Wandler liegt bei 15 MHz mit einem Abtastwert für jeden Wandler von 2 Bit. Hieraus ergibt sich ebenfalls eine Datenrate von 4 GB/s, die in einem solchen Fall durch die Schnittstelle IF in Richtung hin zum Hochfrequenzsender TR mindestens bereitzustellen ist.

Die im Vorangegangenen beschriebene Ausführungsform der Erfindung weist eine Reihe von Vorteilen auf. Insbesondere verarbeitet ein zentrales Rechenwerk in der Form eines Rechners COM asynchron und entkoppelt von den laufenden Messungen des Schallkopfs die zwischengespeicherten Empfangsdaten blockweise zu Bildern. Die Abwesenheit einer messsynchronen Taktung eines Strahlformers ermöglicht die Implementierung der Strahlformung basierend auf Software mittels Standard-Hardwarekomponenten, die üblicherweise in PCs verbaut sind. In der Software kann dabei die Strahlformung mit der Bildformung zur Erzeugung der Bilder auf dem Display vereint werden.

Die Zwischenspeicherung der digitalisierten Rohdaten in einem Pufferspeicher ermöglicht den mehrmaligen Rückgriff und damit eine sequentielle Rekonstruktion einer vollständigen Bildinformation eines Gewebesektors, d.h. für eine Vielzahl von Winkelrichtungen und Gewebetiefen. Somit kann aus einem einzelnen Messdatensatz ein vollständiges zwei- oder ggf. auch dreidimensionales Bild ohne eine Vervielfachung von Strahlformungs-Rechenwerten erreicht werden.

Darüber hinaus erfolgt die oben beschriebene Bildrekonstruktion aus Daten eines gleitenden Zeitintervalls. Hierdurch kann eine weitestgehend konstante Bildrate erreicht werden, die unabhängig von der Zeitsteuerung der parallel mit dem Schallkopf durchgeführten Messungen ist. Vorteilhaft ist eine Bildrate oberhalb der Flickerfrequenz des menschlichen Auges, wie bereits oben erwähnt wurde. Ferner wird vorzugsweise ein synchronisierbares Display zur Anzeige der Bildfolge verwendet, um kurzfristige Schwankungen der Bildrate ausgleichen zu können.

## Patentansprüche

1. Ultraschallvorrichtung zur medizinischen Untersuchung mittels Ultraschallwellen, wobei
- die Ultraschallvorrichtung einen beweglichen Schallkopf (TP) umfasst, der an einen Körper eines Patienten positionierbar ist und ein Wandler-Array (TA) aus elektroakustischen Wandlern umfasst, um Ultraschallsignale in den Körper zu senden und als analoge Rohdaten (RDA) Ultraschallechos der gesendeten Ultraschallsignale zu empfangen;
- der Schallkopf (TP) einen Analog-Digital-Konverter (CON) umfasst, der dazu konfiguriert ist, in Abhängigkeit von den empfangenen analogen Rohdaten (RDA) digitale Rohdaten (RDD) zu generieren, wobei die digitalen Rohdaten (RDD) Messdatensätze (MD) für zeitlich aufeinander folgende Messzeitintervalle umfassen, wobei ein jeweiliger Messdatensatz (MD) Ultraschallechos aus einem Gewebesektor des Körpers, die aus einem Sendevorgang von einem oder mehreren Ultraschallsignalen durch zumindest einen Wandler im Sendebetrieb resultieren, als Abtastwerte für Abtastzeitpunkte des jeweiligen Messzeitintervalls für eine Mehrzahl von Empfangskanälen aus jeweils zumindest einem Wandler im Empfangsbetrieb umfasst;
- der Schallkopf (TP) über eine digitale Datenschnittstelle (IF) mit einer separaten, nicht zum Schallkopf (TP) gehörigen Rechnereinrichtung (COM) gekoppelt ist und derart konfiguriert ist, dass er die digitalen Rohdaten (RDD) über die digitale Datenschnittstelle (IF) streamt;
- die Rechnereinrichtung (COM) einen Rohdaten-Pufferspeicher (BU) umfasst, in dem die jeweiligen Messdatensätze (MD) der digitalen Rohdaten (RDD) gepuffert werden;
- die Rechnereinrichtung (COM) derart ausgestaltet ist, dass sie für die gepufferten Messdatensätze (MD) jeweils eine digitale Strahlformung durch zeitverzögerte Addition von Abtastwerten durchführt, um Bildwerte für eine Vielzahl von Gewebepositionen in verschiedenen Gewebetiefen und mit mehreren Gewebepositionen für jede Gewebetiefe zu ermitteln und hierdurch ein rekonstruiertes Bild des Gewebesektors zu erhalten, und dass sie basierend auf den rekonstruierten Bildern einen Bildstrom aus aufeinander folgenden rekonstruierten Bildern oder aus daraus berechneten Bildern mit einer vorgegebenen Bildwiederholrate generiert und einem Anzeigemittel (DI) zuführt, das den Bildstrom wiedergibt.

2. Ultraschallvorrichtung nach Anspruch 1, **dadurch gekennzeichnet, dass** der Rohdaten-Pufferspeicher (BU) zur gleichzeitigen Pufferung einer Vielzahl von aufeinander folgenden Messdatensätzen (MD) eingerichtet ist.

3. Ultraschallvorrichtung nach Anspruch 2, wobei die Rechnereinrichtung (COM) derart ausgestaltet ist, dass sie für mehrere der Vielzahl von gepufferten Messdatensätzen (MD) parallel jeweilige rekonstruierte Bilder ermittelt.

4. Ultraschallvorrichtung nach einem der vorhergehenden Ansprüche, **dadurch gekennzeichnet, dass** die Rechnereinrichtung (COM) derart ausgestaltet ist, dass die Bilder des Bildstroms jeweils als eine Mittelung aus mehreren aufeinander folgenden rekonstruierten Bildern berechnet werden.

5. Ultraschallvorrichtung nach einem der vorhergehenden Ansprüche, **dadurch gekennzeichnet, dass** die vorgegebene Bildwiederholrate bei 50 Hz oder mehr liegt.

6. Ultraschallvorrichtung nach einem der vorhergehenden Ansprüche, **dadurch gekennzeichnet, dass** die Rechnereinrichtung (COM) derart ausgestaltet ist, dass sie digitale Steuerdaten (CDD) ermittelt, basierend auf denen der Betrieb der Wandler des Wandler-Arrays (TA) zum Senden von Ultraschallsignalen und zum Empfangen von Ultraschallechos für die aufeinander folgenden Messzeitintervalle festgelegt ist, wobei die Rechnereinrichtung (COM) einen Steuerdaten-Pufferspeicher (BU') umfasst, in dem die digitalen Steuerdaten (CDD) gepuffert werden, und wobei der Schallkopf (TP) derart ausgestaltet ist, dass er über die digitale Datenschnittstelle (IF) oder eine weitere digitale Datenschnittstelle zwischen Schallkopf (TP) und Rechnereinrichtung (COM) die digitalen Steuerdaten (CDD) ausliest und basierend auf diesen ausgelesenen Steuerdaten die Wandler des Wandler-Arrays (TA) ansteuert.

7. Ultraschallvorrichtung nach einem der vorhergehenden Ansprüche, **dadurch gekennzeichnet, dass** die digitale Datenschnittstelle (IF) eine drahtgebundene Datenschnittstelle und/oder eine drahtlose Datenschnittstelle umfasst.

8. Ultraschallvorrichtung nach einem der vorhergehenden Ansprüche, **dadurch gekennzeichnet, dass** die digitale Datenschnittstelle (IF) eine PCI-Express-Schnittstelle umfasst.

9. Ultraschallvorrichtung nach einem der vorhergehenden Ansprüche, **dadurch gekennzeichnet, dass** der Schallkopf (TP) einen Hochspannungssender (TR) umfasst, um Hochspannungssignale zu erzeugen, die dem Wandler-Array (TA) zur Erzeugung von Ultraschallwellen durch einen oder mehrere Wandler im Sendebetrieb über ein Weichen-Array (SW) aus Sende-Empfangs-Weichen zugeführt werden, wobei die Wandler des Wandler-Arrays (TA) durch das Weichen-Array (SW) in den Sendebetrieb oder Empfangsbetrieb geschaltet werden können.

10. Ultraschallvorrichtung nach einem der vorhergehenden Ansprüche, **dadurch gekennzeichnet, dass** der Schallkopf (TP) einen Vorverstärker (PA) umfasst, der dazu eingerichtet ist, die analogen Rohdaten (RDA) vor der Zuführung zum Analog-Digital-Konverter (CON) zu verstärken.

## Claims

1. An ultrasonic apparatus for medical examination using ultrasonic waves, wherein
- the ultrasonic apparatus comprises a movable transducer probe (TP) which can be positioned on a body of a patient and includes a transducer array (TA) of electroacoustic transducers for transmitting ultrasonic signals into the body and receiving as analog raw data (RDA) ultrasonic echoes of the transmitted ultrasonic signals;
- the transducer probe (TP) comprises an analog-to-digital converter (CON) which is configured to generate digital raw data (RDD) in dependence on the received analog raw data (RDA), wherein the digital raw data (RDD) comprise measurement data sets (MD) for temporally consecutive measurement time intervals, wherein a respective measurement data set (MD) comprises ultrasonic echoes from a tissue sector of the body, which result from a transmitting operation of one or more ultrasonic signals by at least one transducer in transmitting mode, as samples for sampling instants of the respective measurement time interval for a plurality of receiving channels from, respectively, at least one transducer in receiving mode;
- the transducer probe (TP) is coupled via a digital data interface (IF) to a separate computer device (COM) not belonging to the transducer probe (TP) and is configured to stream the digital raw data (RDD) via the digital data interface (IF);
- the computer device (COM) comprises a raw data buffer memory (BU) in which the respective measurement data sets (MD) of the digital raw data (RDD) are buffered;
- the computer device (COM) is configured to carry out a respective digital beamforming for the buffered measurement data sets (MD) by time-delayed addition of samples, in order to determine image values for a plurality of tissue locations at different tissue depths and with several tissue locations for each tissue depth, and thereby to obtain a reconstructed image of the tissue sector, and to generate, based on the reconstructed images, an image stream of consecutive reconstructed images or of images calculated therefrom with a predetermined image refresh rate and to supply it to a display means (DI) which reproduces the image stream.

2. The ultrasonic apparatus according to claim 1, **characterized in that** the raw data buffer memory (BU) is arranged for the simultaneous buffering of a plurality of consecutive measurement data sets (MD).

3. The ultrasonic apparatus according to claim 2, wherein the computer device (COM) is configured to determine respective reconstructed images in parallel for several of the plurality of buffered measurement data sets (MD).

4. The ultrasonic apparatus according to one of the preceding claims, **characterized in that** the computer device (COM) is configured such that the images of the image stream are each calculated as an averaging of a plurality of consecutive reconstructed images.

5. The ultrasonic apparatus according to one of the preceding claims, **characterized in that** the predetermined image refresh rate is 50 Hz or more.

6. The ultrasonic apparatus according to one of the preceding claims, **characterized in that** the computer device (COM) is configured to determine digital control data (CDD) based on which the operation of the transducers of the transducer array (TA) for transmitting ultrasonic signals and for receiving ultrasonic echoes is determined for the consecutive measurement time intervals, wherein the computer device (COM) comprises a control data buffer memory (BU') in which the digital control data (CDD) are buffered, and wherein the transducer probe (TP) is configured to read the digital control data (CDD) via the digital data interface (IF) or an additional digital data interface between the transducer probe (TP) and the computer device (COM) and, based on these read-out control data, to control the transducers of the transducer array (TA).

7. The ultrasonic apparatus according to one of the preceding claims, **characterized in that** the digital data interface (IF) comprises a wired data interface and/or a wireless data interface.

8. The ultrasonic apparatus according to one of the preceding claims, **characterized in that** the digital data interface (IF) comprises a PCI Express interface.

9. The ultrasonic apparatus according to one of the preceding claims, **characterised in that** the transducer probe (TP) comprises a high voltage transmitter (TR) for generating high voltage signals which are supplied via a switch array (SW) of transmit-receive switches to the transducer array (TA) for generating ultrasonic waves by one or more transducers in transmitting mode, wherein the transducers of the transducer array (TA) can be switched by the switch array (SW) into the transmitting mode or the receiving mode.

10. The ultrasonic apparatus according to one of the preceding claims, **characterized in that** the transducer probe (TP) comprises a preamplifier (PA) configured to amplify the analog raw data (RDA) before supplying it to the analog-to-digital converter (CON).

## Revendications

1. Dispositif à ultrasons pour l'examen médical au moyen d'ondes ultrasonores, dans lequel
- le dispositif à ultrasons comprend une tête émettrice mobile (TP) qui peut être positionnée sur un corps d'un patient et comprend un réseau (TA) de transducteurs électroacoustiques pour transmettre des signaux ultrasonores dans le corps et pour recevoir des échos ultrasonores des signaux ultrasonores transmis en tant que données brutes analogiques (RDA) ;
- la tête émettrice (TP) comprend un convertisseur analogique-numérique (CON) qui est configuré pour générer des données brutes numériques (RDD) en fonction des données brutes analogiques reçues (RDA), dans lequel les données brutes numériques (RDD) comprennent des ensembles de données de mesure (MD) pour des intervalles temporels de mesure successifs dans le temps, dans lequel un ensemble de données de mesure respectif (MD) comprend des échos ultrasonores provenant d'un secteur de tissu du corps qui résultent d'un processus de transmission d'un ou plusieurs signaux ultrasonores par au moins un transducteur en mode de transmission, en tant que valeurs de balayage pour des instants de balayage de l'intervalle temporel de mesure respectif pour une pluralité de canaux de réception provenant à chaque fois d'au moins un transducteur en mode de réception ;
- la tête émettrice (TP) est couplée par le biais d'une interface de données numérique (IF) à un dispositif informatique séparé (COM) n'appartenant pas à la tête émettrice (TP) et configurée de telle sorte qu'elle lit en continu les données brutes numériques (RDD) par le biais de l'interface de données numérique (IF) ;
- le dispositif informatique (COM) comprend une mémoire tampon de données brutes (BU) dans laquelle les ensembles de données de mesure respectifs (MD) des données brutes numériques (RDD) sont mis en mémoire tampon ;
- le dispositif informatique (COM) est élaboré de telle sorte qu'il exécute pour les ensembles de données de mesure mis en mémoire tampon (MD) à chaque fois une formation de faisceau numérique par addition décalée dans le temps de valeurs de balayage pour déterminer des valeurs d'image pour une pluralité de positions de tissu dans différentes profondeurs de tissu et avec plusieurs positions de tissu pour chaque profondeur de tissu, et pour obtenir ce faisant une image reconstruite du secteur de tissu, et qu'il génère en se basant sur les images reconstruites un flux d'images constitué d'images reconstruites successives ou d'images calculées à partir de celles-ci avec un taux de répétition d'image prédéfini et l'achemine à un moyen d'affichage (DI) qui restitue le flux d'images.

2. Dispositif à ultrasons selon la revendication 1, **caractérisé en ce que** la mémoire tampon de données brutes (BU) est configurée pour la mise en mémoire tampon simultanée d'une pluralité d'ensembles de données de mesure successifs (MD).

3. Dispositif à ultrasons selon la revendication 2, dans lequel le dispositif informatique (COM) est élaboré de telle sorte qu'il détermine parallèlement pour plusieurs de la pluralité d'ensembles de données de mesure mis en mémoire tampon (MD) des images reconstruites respectives.

4. Dispositif à ultrasons selon une des revendications précédentes, **caractérisé en ce que** le dispositif informatique (COM) est élaboré de telle sorte que les images du flux d'images sont calculées chacune en tant qu'une moyenne de plusieurs images reconstruites successives.

5. Dispositif à ultrasons selon une des revendications précédentes, **caractérisé en ce que** le taux de répétition d'image prédéfini se situe à 50 Hz ou plus.

6. Dispositif à ultrasons selon une des revendications précédentes, **caractérisé en ce que** le dispositif informatique (COM) est élaboré de telle sorte qu'il détermine des données de commande numériques (CDD) sur la base desquelles le fonctionnement des transducteurs du réseau de transducteurs (TA) est fixé pour la transmission de signaux ultrasonores et pour la réception d'échos ultrasonores pour les intervalles temporels de mesure successifs, dans lequel le dispositif informatique (COM) comprend une mémoire tampon de données de commande (BU') dans laquelle les données de commande numériques (CDD) sont mises en mémoire tampon, et dans lequel la tête émettrice (TP) est élaborée de telle sorte qu'elle lit les données de commande numériques (CDD) par le biais de l'interface de données numérique (IF) ou d'une autre interface de données numérique entre la tête émettrice (TP) et le dispositif informatique (COM), et commande les transducteurs du réseau de transducteurs (TA) en se basant sur ces données de commande lues.

7. Dispositif à ultrasons selon une des revendications précédentes, **caractérisé en ce que** l'interface de données numérique (IF) comprend une interface de données filaire et/ou une interface de données sans fil.

8. Dispositif à ultrasons selon une des revendications précédentes, **caractérisé en ce que** l'interface de données numérique (IF) est une interface PCI-Express.

9. Dispositif à ultrasons selon une des revendications précédentes, **caractérisé en ce que** la tête émettrice (TP) comprend un émetteur haute tension (TR) pour générer des signaux haute tension qui sont acheminés au réseau de transducteurs (TA) pour la génération d'ondes ultrasonores par un ou plusieurs transducteurs en mode de transmission par le biais d'un réseau de filtres séparateurs (SW) constitué de filtres séparateurs de transmission-réception, dans lequel les transducteurs du réseau de transducteurs (TA) peuvent être commutés par le réseau de filtres séparateurs (SW) en mode de transmission ou mode de réception.

10. Dispositif à ultrasons selon une des revendications précédentes, **caractérisé en ce que** la tête émettrice (TP) comprend un préamplificateur (PA) qui est configuré pour renforcer les données brutes analogiques (RDA) avant l'acheminement au convertisseur analogique-numérique (CON).
